# EUROPEAN PATENT SPECIFICATION

(11) **EP 2 135 093 B1**
(45) Date of publication and mention of the grant of the patent: **15.04.2015**
(21) Application number: 08745859.2
(22) Date of filing: 15.04.2008
(51) Int. Cl.: G01N 33/68

(54) **ANALYSIS OF PHOSPHORYLATED GLYCANS, GLCOPEPTIDES OR GLYCOPROTEINS BY IMAC**
ANALYSE PHOSPHORYLIERTER GLYCANE, GLYCOPEPTIDE ODER GLYCOPROTEINE DURCH IMAC
ANALYSE DE GLYCANES, GLYCOPEPTIDES OU GLYCOPROTÉINES PHOSPHORYLÉS PAR IMAC

(30) Priority: 16.04.2007 US 923680 P
(43) Date of publication of application: 23.12.2009
(73) Proprietor: Momenta Pharmaceuticals, Inc., Cambridge, MA 02142 (US)
(72) Inventor: BOSQUES, Carlos, J., Arlington, MA 02476 (US); THIRUNEELAKANTAPILLAI, Lakshmanan, Boston, MA 02125 (US); PARSONS, Ian, Christopher, Belmont, MA 02478 (US)
(74) Representative: Simpson, Tobias Rutger
(86) International application number: PCT/US2008/060337
(87) International publication number: WO 2008/128222

(56) References cited:
- WO-A-2005/107491
- WO-A-2005/123954
- WO-A-2006/014424
- US-A1- 2006 115 863
- AZARKAN ET AL: "Affinity chromatography: A useful tool in proteomics studies" JOURNAL OF CHROMATOGRAPHY B: BIOMEDICAL SCIENCES & APPLICATIONS, ELSEVIER, AMSTERDAM, NL, vol. 849, no. 1-2, 7 April 2007 (2007-04-07), pages 81-90, XP022024428 ISSN: 1570-0232
- POSEWITZ M C ET AL: "Immobilized gallium(III) affinity chromatography of phosphopeptides" ANALYTICAL CHEMISTRY, AMERICAN CHEMICAL SOCIETY. COLUMBUS, US, vol. 71, no. 14, 15 July 1999 (1999-07-15), pages 2883-2892, XP002260363 ISSN: 0003-2700 cited in the application
- KINOSHITA EIJI ET AL: "Novel immobilized zinc(II) affinity chromatography for phosphopeptides and phosphorylated proteins" JOURNAL OF SEPARATION SCIENCE, vol. 28, no. 2, February 2005 (2005-02), pages 155-162, XP002486667 ISSN: 1615-9306
- SLEAT DAVID E ET AL: "Identification and validation of mannose 6-phosphate glycoproteins in human plasma reveal a wide range of lysosomal and non-lysosomal proteins" MOLECULAR & CELLULAR PROTEOMICS, vol. 5, no. 10, October 2006 (2006-10), pages 1942-1956, XP002486668 ISSN: 1535-9476
- SLEAT ET AL: "The human urine mannose 6-phosphate glycoproteome" BIOCHIMICA ET BIOPHYSICA ACTA (BBA) - PROTEINS & PROTEOMICS, ELSEVIER, vol. 1774, no. 3, 8 March 2007 (2007-03-08), pages 368-372, XP005918208 ISSN: 1570-9639
- ZHAO JIA ET AL: "Glycoprotein microarrays with multi-lectin detection: Unique lectin binding patterns as a tool for classifying normal, chronic pancreatitis and pancreatic cancer sera" JOURNAL OF PROTEOME RESEARCH, vol. 6, no. 5, 12 April 2007 (2007-04-12), pages 1864-1874, XP002486669 ISSN: 1535-3893

## Description

### Background

Phosphorylation of glycans can have a significant impact on the biology of glycoconjugates (e.g., glycoproteins) containing the glycans. For example, N-linked glycans containing mannose-6-phosphate (Man-6-P) residues can act as targeting signals for the transport of proteins from the Golgi apparatus to lysosomes. Phosphorylated glycans are commonly found in glycoproteins from many different sources such as therapeutic glycoproteins and glycoproteins found in body fluids, tissues and cells. Sulfation is another common glycan modification that can significantly affect the biological function of glycoconjugates (e.g., glycoproteins). However, phosphate and sulfate groups have essentially the same mass (80 Da). As a result, it is often difficult to differentiate between sulfated and phosphorylated species with traditional mass spectrometric methods. The analysis of phosphorylated glycans is further complicated by their instability. These challenges are magnified when the phosphorylated glycans are part of a complex mixture.

There is a need, therefore, for the development of techniques for analyzing phosphorylated glycan species, and a particular need for techniques that can distinguish between sulfated and phosphorylated species.

### Summary

According to a first aspect of the present invention there is provided a method of separating at least two glycans present in a glycan preparation, the method comprising steps of: (i) providing a glycan preparation that includes at least two glycans with differences in phosphorylation, wherein the at least two glycans have each been cleaved or otherwise released from a glycoconjugate; (ii) contacting the glycan preparation with a resin to which metal ions are immobilized under conditions that cause the at least two glycans to exhibit differing affinities for the resin as a result of the differences in phosphorylation; and (iii) separating the at least two glycans based on their differing affinities for the resin.

According to a second aspect of the present invention there is provided a method of analyzing a sample containing glycans, the method comprising steps of cultivating a cell that produces a plurality of glycoconjugates; obtaining a glycan preparation from said plurality of glycoconjugates, wherein the glycan preparation includes glycans having differences in phosphorylation and wherein said glycans have been cleaved or otherwise released from said plurality of glycoconjugates; and analyzing a sample containing the glycans by contacting the sample with a resin to which metal ions have been immobilized, the contacting being performed under conditions such that individual glycans are differentially retained on the resin as a result of the differences in phosphorylation.

More generally the present disclosure provides technologies for enriching, detecting, and/or quantifying phosphorylated glycans and/or glycoconjugates (e.g., glycopeptides, glycoproteins, glycolipids, proteoglycans, etc.). Such glycans and/or glycoconjugates may be free or may be in the context of larger entities including, for example, cells. Specifically, the present disclosure provides methods that utilize immobilized metal affinity chromatography (IMAC) to isolate and/or to characterize phosphorylated glycans.

In some instances, the present disclosure provides methods of producing a glycoprotein product by cultivating cells that produce a glycoprotein and using IMAC to enrich for or deplete cells producing at least one particular glycoform. In some instances, the present disclosure provides methods of producing a glycoprotein product by obtaining a glycoprotein preparation and using IMAC to enrich for or deplete at least one particular glycoform.

Methods of the present disclosure may be utilized to compare different cultures and/or glycan or glycoconjugate preparations, and/or may be used to assess or improve quality of such cultures or preparations.

### Brief Description of the Drawing

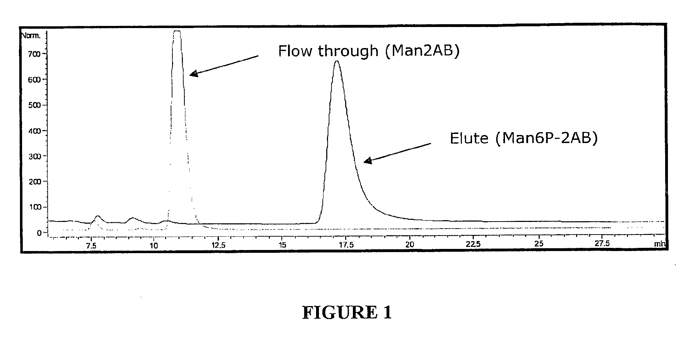

*Figure 1* shows the results of an IMAC separation of a 1:1 mixture of mannose and mannose-6-phosphate applied to a Ga³⁺ IMAC column. Mannose did not bind to the resin while phosphorylated mannose was retained.

### Definitions

*Approximately, About, Ca.*: As used herein, the terms "approximately", "about" or "ca.," as applied to one or more values of interest, refer to a value that is similar to a stated reference value. In certain instances, the terms "approximately", "about" or "ca.," refer to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less of the stated reference value.

*Biological sample:* The term "biological sample", as used herein, refers to any solid or fluid sample obtained from, excreted by or secreted by any living cell or organism, including, but not limited to, tissue culture, bioreactor sample, human or animal tissue, plants, fruits, vegetables, single-celled microorganisms (such as bacteria and yeasts) and multicellular organisms. For example, a biological sample can be a biological fluid obtained from, e.g., blood, plasma, serum, urine, bile, seminal fluid, cerebrospinal fluid, aqueous or vitreous humor, or any bodily secretion, a transudate, an exudate (e.g., fluid obtained from an abscess or any other site of infection or inflammation), or fluid obtained from a joint (e.g., a normal joint or a joint affected by disease such as a rheumatoid arthritis, osteoarthritis, gout or septic arthritis). A biological sample can also be, e.g., a sample obtained from any organ or tissue (including a biopsy or autopsy specimen), can comprise cells (whether primary cells or cultured cells), medium conditioned by any cell, tissue or organ, tissue culture.

*Cell-surface glycoprotein:* As used herein, the term "cell-surface glycoprotein" refers to a glycoprotein, at least a portion of which is present on the exterior surface of a cell. In some instances, a cell-surface glycoprotein is a protein that is positioned on the cell surface such that at least one of the glycan structures is present on the exterior surface of the cell.

*Cell-surface glycan:* A "cell-surface glycan" is a glycan that is present on the exterior surface of a cell. In many instances of the present disclosure, a cell-surface glycan is covalently linked to a polypeptide as part of a cell-surface glycoprotein. A cell-surface glycan can also be linked to a cell membrane lipid.

*Glycan:* As is known in the art and used herein "glycans" are sugars. Glycans can be monomers or polymers of sugar residues, but typically contain at least three sugars, and can be linear or branched. A glycan may include natural sugar residues (e.g., glucose, N-acetylglucosamine, N-acetyl neuraminic acid, galactose, mannose, fucose, hexose, arabinose, ribose, xylose, etc.) and/or modified sugars (e.g., 2'-fluororibose, 2'-deoxyribose, phosphomannose, 6'sulfo N-acetylglucosamine, etc). The term "glycan" includes homo and heteropolymers of sugar residues. The term "glycan" also encompasses a glycan component of a glycoconjugate (e.g., of a glycoprotein, glycolipid, proteoglycan, etc.). The term also encompasses free glycans, including glycans that have been cleaved or otherwise released from a glycoconjugate.

*Glycan preparation:* The term "glycan preparation" as used herein refers to a set of glycans obtained according to a particular production method. In some instances, glycan preparation refers to a set of glycans obtained from a glycoprotein preparation (see definition of glycoprotein preparation below).

*Glycoconjugate:* The term "glycoconjugate", as used herein, encompasses all molecules in which at least one sugar moiety is covalently linked to at least one other moiety. The term specifically encompasses all biomolecules with covalently attached sugar moieties, including for example N-linked glycoproteins, O-linked glycoproteins, glycolipids, proteoglycans, etc.

*Glycoform:* The term "glycoform", is used herein to refer to a particular form of a glycoconjugate. That is, when the same backbone moiety (e.g., polypeptide, lipid, etc) that is part of a glycoconjugate has the potential to be linked to different glycans or sets of glycans, then each different version of the glycoconjugate (i.e., where the backbone is linked to a particular set of glycans) is referred to as a "glycoform".

*Glycolipid:* The term "glycolipid" as used herein refers to a lipid that contains one or more covalently linked sugar moieties (i.e., glycans). The sugar moiety(ies) may be in the form of monosaccharides, disaccharides, oligosaccharides, and/or polysaccharides. The sugar moiety(ies) may comprise a single unbranched chain of sugar residues or may be comprised of one or more branched chains. In certain instances, sugar moieties may include sulfate and/or phosphate groups. In certain instances, glycoproteins contain O-linked sugar moieties; in certain instances, glycoproteins contain N-linked sugar moieties.

*Glycoprotein:* As used herein, the term "glycoprotein" refers to a protein that contains a peptide backbone covalently linked to one or more sugar moieties (i.e., glycans). As is understood by those skilled in the art, the peptide backbone typically comprises a linear chain of amino acid residues. In certain instances, the peptide backbone spans the cell membrane, such that it comprises a transmembrane portion and an extracellular portion. In certain instances, a peptide backbone of a glycoprotein that spans the cell membrane comprises an intracellular portion, a transmembrane portion, and an extracellular portion. In certain instances, methods of the present disclosure comprise cleaving a cell surface glycoprotein with a protease to liberate the extracellular portion of the glycoprotein, or a portion thereof, wherein such exposure does not substantially rupture the cell membrane. The sugar moiety(ies) may be in the form of monosaccharides, disaccharides, oligosaccharides, and/or polysaccharides. The sugar moiety(ies) may comprise a single unbranched chain of sugar residues or may comprise one or more branched chains. In certain instances, sugar moieties may include sulfate and/or phosphate groups. Alternatively or additionally, sugar moieties may include acetyl, glycolyl, propyl or other alkyl modifications. In certain instances, glycoproteins contain O-linked sugar moieties; in certain instances, glycoproteins contain N-linked sugar moieties. In certain instances, methods disclosed herein comprise a step of analyzing any or all of cell surface glycoproteins, liberated fragments (e.g., glycopeptides) of cell surface glycoproteins, cell surface glycans attached to cell surface glycoproteins, peptide backbones of cell surface glycoproteins, fragments of such glycoproteins, glycans and/or peptide backbones, and combinations thereof.

*Glycosidase:* The term "glycosidase" as used herein refers to an agent that cleaves a covalent bond between sequential sugars in a glycan or between the sugar and the backbone moiety (e.g., between sugar and peptide backbone of glycoprotein). In some instances, a glycosidase is an enzyme. In certain instances, a glycosidase is a protein (e.g., a protein enzyme) comprising one or more polypeptide chains. In certain instances, a glycosidase is a chemical cleavage agent.

*Glycosylation pattern:* As used herein, the term "glycosylation pattern" refers to the set of glycan structures present on a particular sample. For example, a particular glycoconjugate (e.g., glycoprotein) or set of glycoconjugates (e.g., set of glycoproteins) will have a glycosylation pattern. In some instances, reference is made to the glycosylation pattern of cell surface glycans. A glycosylation pattern can be characterized by, for example, the identities of glycans, amounts (absolute or relative) of individual glycans or glycans of particular types, degree of occupancy of glycosylation sites, etc., or combinations of such parameters.

*Glycoprotein preparation:* A "glycoprotein preparation", as that term is used herein, refers to a set of individual glycoprotein molecules, each of which comprises a polypeptide having a particular amino acid sequence (which amino acid sequence includes at least one glycosylation site) and at least one glycan covalently attached to the at least one glycosylation site. Individual molecules of a particular glycoprotein within a glycoprotein preparation typically have identical amino acid sequences but may differ in the occupancy of the at least one glycosylation sites and/or in the identity of the glycans linked to the at least one glycosylation sites. That is, a glycoprotein preparation may contain only a single glycoform of a particular glycoprotein, but more typically contains a plurality of glycoforms. Different preparations of the same glycoprotein may differ in the identity of glycoforms present (e.g., a glycoform that is present in one preparation may be absent from another) and/or in the relative amounts of different glycoforms.

*Isotopically labeled:* As used herein, an "isotopically labeled" agent (which may also possess properties such as a fluorophore, a heterobifunctional ligand, or a chromophore) is a reagent which bears an atom or set of atoms which have been isotopically enriched by chemical or enzymatic methods. For example, an isotopically labeled agent may be isotopically enriched by direct replacement of an atom, either by chemical or enzymatic methods, with an isotopic label (e.g., a proton (¹H) with a deuterium (²H) atom or atoms; a proton (¹H) with a tritium (³H) atom or atoms; a ¹²C with a ¹³C atom or atoms; a ¹⁶O with a ¹⁸O atom or atoms; a ¹⁴N with a ¹⁵N atom or atoms; a ³¹P with a ³²P atom or atoms, and the like), or by chemical synthesis. Exemplary isotopic labels include, but are not limited to, ²H, ³H, ¹³C, ¹⁸O, ¹⁵N, ¹⁸O, ³³S, ³⁴S, ³²P, ²⁹Si, and ³⁰Si. Furthermore, a glycan preparation may be "isotopically labeled" by reaction with an isotopically labeled agent (i.e., the reaction transfers an isotopic label to the glycan moiety).

*N-glycan:* The term "N-glycan," as used herein, refers to a polymer of sugars that has been released from a glycoconjugate but was formerly linked to the glycoconjugate via a nitrogen linkage (see definition of N-linked glycan below).

*N-linked glycans:* N-linked glycans are glycans that are linked to a glycoconjugate via a nitrogen linkage. A diverse assortment of N-linked glycans exists, but is typically based on the common core pentasaccharide (Man)₃(GlcNAc)(GlcNAc).

*O-glycan:* The term "O-glycan," as used herein, refers to a polymer of sugars that has been released from a glycoconjugate but was formerly linked to the glycoconjugate via an oxygen linkage (see definition of O-linked glycan below).

*O-linked glycans:* O-linked glycans are glycans that are linked to a glycoconjugate via an oxygen linkage. O-linked glycans are typically attached to glycoproteins via N-acetyl-D-galactosamine (GalNAc) or via N-acetyl-D-glucosamine (GlcNAc) to the hydroxyl group of L-serine (Ser) or L-threonine (Thr). Some O-linked glycans also have modifications such as acetylation and sulfation. In some instances O-linked glycans are attached to glycoproteins via fucose or mannose to the hydroxyl group of L-serine (Ser) or L-threonine (Thr).

*Phosphorylation:* As used herein, the term "phosphorylation" refers to the process of covalently adding one or more phosphate groups to a molecule (e.g., to a glycan).

*Protease:* The term "protease" as used herein refers to an agent that cleaves a peptide bond between sequential amino acids in a polypeptide chain. In some instances, a protease is an enzyme (i.e., a proteolytic enzyme). In certain instances, a protease is a protein (e.g., a protein enzyme) comprising one or more polypeptide chains. In certain instances, a protease is a chemical cleavage agent.

*Protein:* In general, a "protein" is a polypeptide (i.e., a string of at least two amino acids linked to one another by peptide bonds). Proteins may include moieties other than amino acids (e.g., may be glycoproteins) and/or may be otherwise processed or modified. Those of ordinary skill in the art will appreciate that a "protein" can be a complete polypeptide chain as produced by a cell (with or without a signal sequence), or can be a functional portion thereof. Those of ordinary skill will further appreciate that a protein can sometimes include more than one polypeptide chain, for example linked by one or more disulfide bonds or associated by other means.

*Resin:* As used herein, a "resin" is a solid phase. In many instances, a resin is comprised of a organic polymer. The polymer may be naturally occurring or synthetic.

*Sialic acid:* The term "sialic acid," as used herein, is a generic term for the *N*or *O*-substituted derivatives of neuraminic acid, a nine-carbon monosaccharide. The amino group of neuraminic acid typically bears either an acetyl or a glycolyl group in a sialic acid. The hydroxyl substituents present on the sialic acid may be modified by acetylation, methylation, sulfation, and phosphorylation. The predominant sialic acid is N-acetylneuraminic acid (Neu5Ac). Sialic acids impart a negative charge to glycans, because the carboxyl group tends to dissociate a proton at physiological pH. Exemplary deprotonated sialic acids are as follows:

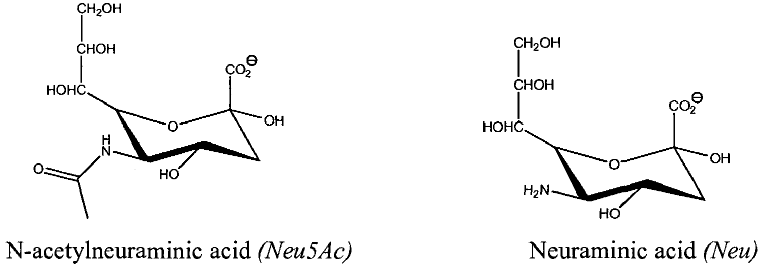

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena. To give but one particular example, when it is said that a treatment does not "substantially" rupture the cell membranes, it is meant to indicate that all or most of the cell membranes remain intact during and after the treatment, for example so that intracellular glycoproteins or glycopeptides are thus not released from the cells. In certain instances, the term "substantially", as applied to unruptured cell membranes, refers to condition wherein 15%, 10%, 9%, 8%, 7%, 6%, 5%, 4%, 3%, 2%, 1%, or fewer of the cells subjected to a particular treatment exhibit measurable ruptured cell membranes. In certain instances, the term "substantially", as applied to unruptured cell membranes, refers to condition wherein none of the cells subjected to a particular treatment exhibit measurable ruptured cell membranes.

### Detailed Description of Certain Embodiments

The present disclosure provides technologies for enriching, detecting, and/or quantifying phosphorylated glycans and/or glycoconjugates (e.g., glycopeptides, glycolipids, proteoglycans, etc.). Specifically, the present disclosure provides methods that utilize immobilized metal affinity chromatography (IMAC) to isolate and/or to characterize phosphorylated glycans. The methods are useful, inter alia, in the characterization, manufacture, or quality control of glycoprotein products.

### Glycan Phosphorylation

In general, the hydroxyl groups of the component saccharides of a glycan are susceptible to a number of modifications including phosphorylation. Phosphorylation most often occurs at the C-6 position of mannose in high-mannose N-glycan structures. N-linked glycans containing mannose-6-phosphate (Man-6-P) residues play a key role as targeting signals for the transport of proteins from the Golgi apparatus to lysosomes.

Specifically, lysosomal proteins that receive a Man-6-P modification in the Golgi bind to Man-6-P receptors and are transported to pre-lysosomal compartments, where they are released due to the acidic environment of the compartment. Many of these proteins apparently are associated with metabolic processes; their mutation results in development of lysosomal storage disorders (see, for example, Scriver et al., The Metabolic & Molecular Bases of Inherited Disease, 8th Ed., Vol. III, pp. 134-3894, McGraw-Hill, New York, 2001). In addition, researchers have proposed that phosphorylated lysosomal glycoproteins may be involved in a variety of other pathogenic processes, including tumor invasion and metastasis, Alzheimer's Disease, rheumatoid arthritis, atherosclerosis, etc. (see, for example, Nixon et al., Neurochem. Res. 25:1161, 2000; Bromme et al., Curr. Pharm. Des. 8:1639, 2002; Du et al., Curr. Opin. Lipidol. 15:539, 2004; Fehrenbacher et al., Cancer Res. 65:2993, 2005).

The present disclosure, therefore, by providing improved methods for detecting, enriching, isolating, and/or quantifying phosphorylated glycans provides, among other things, strategies for detecting susceptibility to or incidence of one or more diseases or disorders associated with lysosomal glycoprotein trafficking.

### Immobilized Metal Affinity Chromatography

Immobilized Metal Affinity Chromatography (IMAC) was developed as a technique for separating proteins based on affinity differences for immobilized metal ions. Typically, a transition metal ion (e.g., Al³⁺, Co²⁺, Cu⁺, Cu²⁺, Fe³⁺, Ga³⁺, Ni²⁺, Zn²⁺, Zr³⁺, and/or titanium oxide) is attached to suitable support such as a resin (e.g., resin beads in a column). A sample of proteins is then contacted with the resin (e.g., by flowing over the resin), and proteins in the sample that have affinity for the immobilized metal ion are retained on the resin, while others are not. Proteins that lack affinity for a metal ion can be engineered to include a region with appropriate affinity (e.g., a hexa-histidine tail which binds strongly to Ni²⁺). Retained proteins are then eluted off of the resin, for example, by changing the pH, and/or by adding a specific molecule, such as imidazole that competes for binding to the immobilized metal ions.

The present disclosure is based, in part, on our observation that IMAC can be used to separate glycans based on differences in phosphorylation. Thus, in one instance, the present disclosure provides methods for separating two or more glycans based on differences in phosphorylation. The methods comprise steps of (i) providing a glycan preparation that includes at least two glycans with differences in phosphorylation; (ii) contacting the glycan preparation with a resin to which metal ions are immobilized under conditions that cause the at least two glycans to exhibit differing affinities for the resin as a result of the differences in phosphorylation; and (iii) separating the at least two glycans based on their differing affinities for the resin.

The glycan preparation includes at least two glycans with differences in phosphorylation. For example, the glycan preparation may include a non-phosphorylated glycan and a phosphorylated glycan. The glycan preparation may alternatively include two glycans that are both phosphorylated but in different manners, e.g., because the phosphate groups are at different locations within the glycans and/or because the glycans include different numbers of phosphate groups.

It is to be understood that the phosphorylation may occur anywhere within the glycan(s). Most commonly, phosphorylation will occur at the C-6 position of a mannose moiety.

According to the present disclosure, a glycan preparation is contacted with a resin to which metal ions are immobilized. In one instance, the metal ions are transition metal ions (e.g., Al³⁺, Co²⁺, Cu⁺, Cu²⁺, Fe³⁺, Ga³⁺, Ni²⁺, Zn²⁺, Zr³⁺, and/or titanium oxide). In one instance, the metal ions comprise Cu⁺, Ga³⁺ and/or Zn²⁺. In one instance, the metal ions comprise Cu⁺. In one instance, the metal ions comprise Ga³⁺. In one instance, the metal ions comprise Zn²⁺. In one instance, the metal ions comprise titanium oxide.

Any known IMAC resin may be used according to the present disclosure (e.g., see reviews by Sun et al., Expert Rev. Proteomics 2:649-657, 2005 and Kange et al., J. Biomol. Tech. 16:91-103, 2005 and references cited therein).

In one instance, the resin can be packed into a column (e.g., in the form of beads that have been packed into a column). In another instance, the resin can be in suspension (e.g., in the form of beads in a suspension). Also, in another instance, the resin can be incorporated into a spin column or a multiple-well plate.

A variety of IMAC resins are available commercially and are commonly preloaded with metal ions (e.g., from Bio-Rad Laboratories, Inc. of Hercules, CA; Pierce Biotechnology of Rockford, IL; etc.).

In general, according to the present disclosure, a glycan preparation will be contacted with a resin under conditions that cause the at least two glycans to exhibit differing affinities for the resin. In some instances, the difference in affinities can be attributed to differences in phosphorylation.

These conditions of contact may include the nature of the solvent, buffer, pH, temperature, salt concentration, denaturant(s), etc. In one instance, the glycan preparation is contacted with the resin in the form of an aqueous solution. A buffer may be present in a glycan preparation. Any buffer may be used; however in certain instances one might use acetic acid, trifluoroacetic acid, hydrochloric acid, ammonium acetate, sodium acetate, sodium bicarbonate, and/or ammonium bicarbonate buffer.

In one instance, the pH may range from about 1 to 12, e.g., about 2 to 10. In one instance, the temperature may range from about 3°C to about 40°C, e.g., about 15°C to about 35°C. In one instance, the salt concentration may range from about 0 to about 1M, e.g., about 10mM to about 100mM.

In one instance, the glycan preparation may include additives, e.g., one or more denaturants or buffers such as NP-40, sodium dodecyl sulfate, dithiothreitol, urea, and citric acid salts.

As a result of their differing affinities for the resin, the at least two glycans will exhibit different retention times on the resin. The glycan with a higher affinity for the resin will exhibit longer retention times than the glycan with a lower affinity for the resin. In one instance, the glycan with a lower affinity will be collected from the resin before the glycan with a higher affinity. As is well known in the art, this can readily be achieved by collecting separate aliquots of the glycan preparation for successive time intervals. Subsequent characterization of each aliquot (e.g., by mass spectroscopy, by fluorescence if the glycans are labelled with a fluorescent tag, etc.) will allow the user to determine which aliquots contained relevant glycans.

### Glycan Preparations

Techniques of the present disclosure can be applied to the analysis of glycan preparations from any source. Among other sources, techniques described herein can be applied to glycan preparations from therapeutic formulations and biological samples. For instance, according to the present disclosure techniques described herein can be applied to any biological sample.

In some instances, a biological sample is subjected to one or more processing steps prior to being processed by IMAC as described herein. For example, in some instances, a biological sample is treated with at least one protease, at least one glycosidase, and/or at least one modification agent prior to being subjected to IMAC. Alternatively or additionally, a biological sample is processed by one or more fractionation or other separation techniques, for example to enrich or deplete the sample of one or more components (including, for example, one or more glycoforms).

In some instances, a biological sample is processed by exposure to one or more proteases so that one or more glycoproteins in the sample are cleaved. In some such instances, the protease is selected from the group consisting of trypsin, chymotrypsin, elastase, subtilisin, proteinase K, pepsin, ficin, bromelin, plasmepsin, renin, chymosin, papain, a cathepsin (e.g. cathepsin K), a caspase (e.g. CASP3, CASP6, CASP7, CASP14), calpain 1, calpain 2, hermolysin, carboxypeptidase A or B, matrix metalloproteinase, a glutamic acid protease, and/or combinations thereof.

In some instances, a biological sample that contains cells is treated with one or more proteases under conditions that avoid disruption of the cellular membrane. In some such instances, at least about 75%, 80%, 85%, 90%, 95% or more of cell membranes remain intact.

In some instances, a biological sample is processed by exposure to one or more glycosidic enzymes (e.g., exoglycodiases) so that one or more glycans in the sample is cleaved. Several examples of useful enzymes are reviewed in R.A. O'Neill, Enzymatic release of oligosaccharides from glycoproteins for chromatographic and electrophoretic analysis. J. Chromatog. A 720:201, 1996 and/or S. Prime, et al., Oligosaccharide sequencing based on exo- and endo-glycosidase digestion and liquid chromatographic analysis of the products. J. Chromatog. A 720:263, 1996. In some instances, a biological sample is processed by exposure to one or more of sialidase, galactosidase, hexosaminidase, fucosidase, and mannosidase. In certain instances, the enzyme PNGase F (Peptide N-Glycosidase F) is used to release glycans. PNGase F is an amidase that cleaves the amide bond between the innermost GlcNAc and asparagine residues of high mannose, hybrid, and complex N-linked oligosaccharides. Alternatively or additionally, in certain instances, one or more of the enzymes PNGase A, O-glycanase, and/or Endo-H is/are used to release glycans.

In some instances, a biological sample is processed by exposure to one of more chemical cleavage agents that remove glycans from glycoconjugates. Exemplary such agents include hydrazine, sodium borohydride and/or trifluoromethanesulfonic acid (TFMS).

In some instances, a biological sample is processed by exposure to a labeling agent so that one or more glycans in the sample is detectably labeled. In some instances, the label emits radiation (e.g., is a chromophore, fluorophore, etc). In some instances, the label acts as a mass tag.

In some instances, a biological sample is processed by modification of one or more free chemical groups on glycans within the sample. For example, in some instances, free carboxylic acid groups are modified, for example, by alkylation, acylation, etc. According to the present disclosure, alkylation of glycans can facilitate their analysis by IMAC.

In some instances, glycan preparations for analysis as described herein are obtained from preparations of glycoproteins. In some instances, glycan and/or glycoprotein preparations are obtained from cell culture and/or from a bioreactor. In some instances, a glycan and/or glycoprotein preparation is isolated from a cell. In certain instances, a glycan and/or glycoprotein preparation is obtained from a cell's surface. In certain instances, a glycan and/or glycoprotein preparation is obtained from a cell's secretion. In certain instances, a glycan and/or glycoprotein preparation is obtained from a lysed cell.

In one instance, methods of the present disclosure provide tools for comparing glycan preparations obtained from different sources. The methods discussed herein are not limited to any specific source or type of glycan preparation. To give but one example, glycan preparations may be obtained from glycoprotein preparations which were obtained from cell culture.

Different glycan and/or glycoprotein preparations may be obtained by using, for example, different cell types to generate the glycoprotein preparation; by varying cell culture conditions; or by obtaining different samples of the same cell culture at different time increments. Alternatively or additionally, different preparations can be prepared by utilizing different steps or conditions of isolation or purification.

Different glycan preparations may also be prepared through differential treatment of the same glycoprotein (or other glycoconjugate) preparations. For instance, different glycan preparations may be obtained through the use of different cleavage, isolation, and/or purification conditions.

In certain instances, a first glycan preparation is obtained from a first glycoprotein preparation. In certain instances, a second glycan preparation is obtained from a second glycoprotein preparation. In certain instances, the first glycoprotein preparation is different from the second glycoprotein preparation. In certain particular instances, the difference between the first and second glycoprotein preparations includes at least one difference in phosphorylation (e.g., in type of glycan that is phosphorylated, in extent or degree of phosphorylation, etc.). In certain instances, first and second glycan preparations are obtained from the same cell preparation. In certain instances, first and second glycan preparations are obtained from different cell preparations.

### Growth of Cells in Cell Culture

Any of a variety of cells and/or cell lines that produce glycans (including cells that produce one or more glycoconjugates, e.g., glycoproteins) can be used to prepare a glycan preparation for analysis in accordance with the present disclosure. Suitable cell lines include, but are not limited to, mammalian cells, avian cells, fish cells, insect cells, plant cells, fungal cells, and hybrid cells.

Exemplary mammalian cell lines that can be used in accordance with the present disclosure include, but are not limited to, Chinese hamster ovary (CHO) cells, HeLa cells, Madin-Darby canine kidney (MDCK) cells, baby hamster kidney (BHK - cells), NSO cells, MCF-7 cells, MDA-MB-438 cells, U87 cells, A172 cells, HL60 cells, A549 cells, SP10 cells, DOX cells, DG44 cells, HEK 293 cells, SHSY5Y, Jurkat cells, BCP-1 cells, COS cells, Vero cells, GH3 cells, 9L cells, 3T3 cells, MC3T3 cells, C3H-10T1/2 cells, NIH-3T3 cells, and C6/36 cells.

Exemplary fish cell lines that can be used in accordance with the present disclosure include, but are not limited to, ZF4 cells, AB9 cells, GAKS cells, OLF-136 cells, CAEP cells, CAF cells, OLHE-131 cells, OLME-104 cells, ULF-23 cells, BRF41 cells, Hepa-E1 cells, Hepa-T1 cells, GEM-81 cells, GEM-199 cells, GEM-218 cells, GAKS cells, D-11 cells, R1 cells, RTG-2 cells, RTO cells, and TPS cells. A more complete list can be found in Fryer and Lannan, 2005, "Three decades of fish cell culture: a current listing of cell lines derived from fishes," J. Tissue Culture Methods, 16:87-94.

Exemplary insect cell lines that can be used in accordance with the present disclosure include, but are not limited to, SFM cells, Sf21 cells, Sf9 cells, Schneider cells, S2 cells, T.ni cells, SES-MaBr-1 cells, SES-MaBr-3 cells, NIAS-MB-25 cells, NIAS-MaBr-92 cells, FRI-SpIm-1229 cells, SES-MaBr-4 cells, NIAS-LeSe-11 cells, TUAT-SpLi-221 cells, NIAS-PX-64 cells, NIAS-MB-32 cells, NIAS-MaBr-93 cells, SES-MaBr-5 cells, BM-N cells, NIAS-PX-58 cells, MBHL-2 cells, and MBHL-3 cells.

Those of ordinary skill in the art will recognize that this is an exemplary, not a comprehensive, listing of various cell lines that may be used in accordance with the present disclosure. Other cell lines may be advantageously utilized to produce a glycoprotein preparation.

Any of a variety of cell culture media, including complex media and/or serum-free culture media, that are capable of supporting growth of the one or more cell types or cell lines may be used in accordance with the present disclosure. Typically, a cell culture medium contains a buffer, salts, energy source, amino acids (e.g., natural amino acids, non-natural amino acids, etc.), vitamins and/or trace elements. Cell culture media may optionally contain a variety of other ingredients, including but not limited to, carbon sources (e.g., natural sugars, non-natural sugars, etc.), cofactors, lipids, sugars, nucleosides, animal-derived components, hydrolysates, hormones/growth factors, surfactants, indicators, minerals, activators/inhibitors of specific enzymes, and organics (e.g., butyrate, which induces apoptosis, which releases glycosylases, often slows down growth rate of cell, which changes glycosyltransferase levels, which can result in more mature glycosylation; and results in change in energy of cell; chloroquin, which affects intracellular pH; betaine, an osmoprotectant; ammonia, which alters intracellular pH levels and which can change glycosyl transferase efficiency; etc.), and/or small molecule metabolites (e.g., CMP-sialic acid, glucosamine, Bertozzi compounds, etc.). Cell culture media suitable for use in accordance with the present disclosure are commercially available from a variety of sources, e.g., ATCC (Manassas, Va).

In certain instances, one or more of the following media are used to grow cells: RPMI-1640 Medium, Dulbecco's Modified Eagle's Medium, Minimum Essential Medium Eagle, F-12K Medium, Iscove's Modified Dulbecco's Medium. As will be understood by those of ordinary skill in the art, when defined medium that is serum-free and/or peptone-free is used, the medium is typically highly enriched for amino acids and trace elements (see, for example, U.S. Pat. No. 5,122,469 to Mather et al., and U.S. Pat. No. 5,633,162 to Keen et al.). Different cell culture media may affect the glycosylation pattern of glycoproteins expressed in that medium. For example, a given cell culture media may result in production of glycoproteins with an increased glycosylation pattern, a decreased glycosylation pattern, or a combination of increased and decreased glycosylation patterns. One of ordinary skill in the art will be aware of and will be able to choose one or more suitable cell culture media for use in growing cells whose cell surface protein-linked glycans are to be analyzed using certain methods of the present disclosure.

In some instances, cells are cultured in batch culture, fed batch culture, perfusion culture, static suspension (e.g., roller bottles, T flasks, microcarriers, T150, etc.), and/or on shakers.

Cells used to generate a glycan or glycoprotein preparation can be grown under any of a variety of cell culture conditions. In some instances, cells are cultured under cell culture conditions such that a given glycoprotein in a glycoprotein preparation exhibits a desired glycosylation pattern. In some instances, one or more cell culture conditions are controlled and/or modified in order to produce glycoproteins (or other glycoconjugates) that exhibit more desirable glycosylation patterns. Such cell culture conditions include, but are not limited to, pH, CO₂ levels, oxygen levels, culture agitation rate, redox conditions, culture temperature, cell density, density of seed culture, duration of culture, reactor design, sparge rate, and/or osmolarity. As will be recognized by those of ordinary skill in the art, however, it will often be useful to predict and/or verify that a produced glycoprotein exhibits a desirable glycan phosphorylation pattern. As such, certain methods of the present disclosure that comprise determining the overall glycan phosphorylation status of a cell by characterizing the glycan phosphorylation pattern of cell surface glycoproteins are useful since such methods permit better prediction and/or control of the glycan phosphorylation pattern of a produced therapeutic glycoprotein.

Any of a variety of methods can be used to purify cells from the cell culture medium. In certain instances, cells are grown in a suspension culture. In such instances, cells may be purified from the cell culture medium by one or more cycles of centrifugation and washing (e.g., with a physiological suitable washing solutions such as phosphate-buffered saline). In many instances, care is taken not to centrifuge the cells with too much force in order to avoid unnecessary cell breakage.

In certain instances, cells are grown in an adhesion culture. In such instances, cells may be purified from the cell culture medium by first releasing them from the culture surface. For example, cells may be released from the culture surface by subjecting them to EDTA. Those of ordinary skill in the art will be aware of other suitable agents that can be used to release adherent cells from the culture surface. After release, cells may be purified by one or more cycles of centrifugation and washing (e.g., with a physiological suitable washing solutions such as phosphate-buffered saline). As with cells grown in suspension culture, care should be taken not to centrifuge the cells with too much force in order to avoid unnecessary cell breakage.

### Applications

It will be appreciated that the techniques described herein can be utilized in any of a variety of applications. For example, any application that involves the analysis of glycan phosphorylation and/or the isolation of phosphorylated glycans from a sample will benefit from the present methods. In some instances, the present disclosure involves comparison of glycan phosphorylation in two or more glycans.

Methods of the present disclosure can be applied to glycans obtained from a wide variety of sources including, but not limited to, therapeutic formulations and biological samples. A biological sample may undergo one or more analysis and/or purification steps prior to or after being analyzed according to the present disclosure. To give but a few examples, in some instances, a biological sample is treated with one or more proteases and/or glycosidases (e.g., so that glycans are released); in some instances, glycans in a biological sample are labeled with one or more detectable markers or other agents that may facilitate analysis by, for example, mass spectrometry or NMR. Any of a variety of separation and/or isolation steps may be applied to a biological sample in accordance with the present disclosure.

Methods of the present disclosure can be utilized to analyze glycans in any of a variety of states including, for instance, free glycans, glycoconjugates (e.g., glycopeptides, glycolipids, proteoglycans, etc.), cell-associated glycans (e.g., nucleus-, cytoplasm-, cell-membrane-associated glycans, etc.); glycans associated with cellular, extracellular, intracellular, and/or subcellular components (e.g., proteins); glycans in extracellular space (e.g., cell culture medium), etc.

Methods of the present disclosure may be used in one or more stages of process development for the production of a therapeutic or other commercially relevant glycoprotein of interest. Non-limiting examples of such process development stages that can employ methods of the present disclosure include cell selection, clonal selection, media optimization, culture conditions, process conditions, and/or purification procedure. Those of ordinary skill in the art will be aware of other process development stages.

The present disclosure can also be utilized to monitor the extent and/or type of glycosylation occurring in a particular cell culture, thereby allowing adjustment or possibly termination of the culture in order, for example, to achieve a particular desired glycosylation pattern or to avoid development of a particular undesired glycosylation pattern. In some instances, the present disclosure can be utilized to compare the extent and/or type of glycosylation occurring in different cell cultures. In some instances, methods in accordance with the disclosure may be used to monitor the glycosylation pattern of glycoproteins produced during the course of their production by cells. For example, production of a glycoprotein (e.g., commercial production) may involve steps of (1) culturing cells that produce the glycoprotein, (2) obtaining samples at regular or irregular intervals during the culturing, and (3) analyzing the glycosylation pattern of produced glycoprotein(s) in obtained samples. In some instances, such methods may comprise a step of comparing the glycosylation patterns of produced glycoproteins in obtained samples to one another. In some instances, such methods may comprise comparing the glycosylation patterns of produced glycoproteins in obtained samples to the glycosylation pattern of a reference sample.

The present disclosure can also be utilized to assess glycosylation characteristics of cells or cell lines that are being considered for production of a particular desired glycoprotein (for example, even before the cells or cell lines have been engineered to produce the glycoprotein, or to produce the glycoprotein at a commercially relevant level).

In some instances of the disclosure, a desired glycosylation pattern for a particular target glycoprotein is known, and the technology described herein allows monitoring of culture samples to assess progress of the production along a route known to produce the desired glycosylation pattern. For example, where the target glycoprotein is a therapeutic glycoprotein, for example having undergone regulatory review in one or more countries, it will often be desirable to monitor cultures to assess the likelihood that they will generate a product with a glycosylation pattern as close to the established glycosylation pattern of the pharmaceutical product as possible, whether or not it is being produced by exactly the same route. As used herein, "close" refers to a glycosylation pattern having at least about a 75%, 80%, 85%, 90%, 95%, 98%, or 99% correlation to the established glycosylation pattern of the pharmaceutical product. In such instances, samples of the production culture are typically taken at multiple time points and are compared with an established standard or with a control culture in order to assess relative glycosylation.

In some instances of the present disclosure, a desired glycosylation pattern will be more extensive. For example, in some instances, a desired glycosylation pattern shows high (e.g., greater than about 60%, about 65%, about 70%, about 75%, about 80%, about 85%, about 90%, about 95%, about 98%, about 99%, or more) occupancy of glycosylation sites; in some instances, a desired glycosylation pattern shows, a high degree of branching (e.g., greater than about 60%, about 65%, about 70%, about 75%, about 80%, about 85%, about 90%, about 95%, about 98%, about 99% or more have tri or tetra-antennary structures).

In some instances of the present disclosure, a desired glycosylation pattern will be less extensive. For example, in some instances, a desired cell surface glycosylation pattern shows low (e.g., less than about 50%, about 45%, about 40%, about 35%, about 30%, about 25%, about 20%, about 15%, about 15%, about 5%, about 1%, or less) occupancy of glycosylation sites; and/or a low degree of branching (e.g., less than about 20%, about 15%, about 10%, about 5%, about 1% or less have tri or tetra-antennary structures).

In some instances, a desired glycosylation pattern will be more extensive in some instances and less extensive in others. For example, it may be desirable to employ a cell line that tends to produce glycoproteins with long, unbranched oligosaccharide chains. Alternatively, it may be desirable employ a cell line that tends to produce glycoproteins with short, highly branched oligosaccharide chains.

In some instances, a desired glycosylation pattern will be enriched for a particular type of glycan structure. For example, in some instances, a desired glycosylation pattern will have low levels (e.g., less than about 20%, about 15%, about 10%, about 5%, about 1%, or less) of high mannose or hybrid structures, high levels (e.g., greater than about 60%, about 65%, about 70%, about 75%, about 80%, about 85%, about 90%, about 95%, about 98%, about 99%, or more) of high mannose structures, high levels (e.g., greater than about 60%, about 65%, about 70%, about 75%, about 80%, about 85%, about 90%, about 95%, about 98%, about 99%, or more; for example at least one per glycoprotein) phosphorylated high mannose, or low levels *(e.g.,* less than about 20%, about 15%, about 10%, about 5%, about 1%, or less) of phosphorylated high mannose.

In some instances, a desired glycosylation pattern will include at least about one sialic acid. In some instances, a desired glycosylation pattern will include a high level (e.g., greater than about 60%, about 65%, about 70%, about 75%, about 80%, about 85%, about 90%, about 95%, about 98%, about 99%, or more) of termini that are sialylated. In some instances, a desired glycosylation pattern that includes sialyation will show at least about 85%, about 90%, about 95%, about 98%, about 99%, or more N-acetylneuraminic acid and/or less than about 20%, about 15%, about 10%, about 5%, about 1%, or less *N*-glycolylneuraminic acid.

In some instances, a desired glycosylation pattern shows specificity of branch elongation (e.g., greater than about 60%, about 65%, about 70%, about 75%, about 80%, about 85%, about 90%, about 95%, about 98%, about 99%, or more of extension is on α1,6 mannose branches; or greater than about 60%, about 65%, about 70%, about 75%, about 80%, about 85%, about 90%, about 95%, about 98%, about 99%, or more of extension is on α1,3 mannose branches).

In some instances, a desired glycosylation pattern will include a low level *(e.g.,* less than about 20%, about 15%, about 10%, about 5%, about 1%, or less) or high level (e.g., greater than about 60%, about 65%, about 70%, about 75%, about 80%, about 85%, about 90%, about 95%, about 98%, about 99%, or more) of core fucosylation.

In some instances, a desired glycosylation pattern will include a low level *(e.g.,* less than about 20%, about 15%, about 10%, about 5%, about 1%, or less) or high level (e.g., greater than about 60%, about 65%, about 70%, about 75%, about 80%, about 85%, about 90%, about 95%, about 98%, about 99%, or more) of a sulfated glycan.

In some instances, a desired glycosylation pattern will include a low level *(e.g.,* less than about 20%, about 15%, about 10%, about 5%, about 1%, or less) or high level (e.g., greater than about 60%, about 65%, about 70%, about 75%, about 80%, about 85%, about 90%, about 95%, about 98%, about 99%, or more) of a phosphorylated glycan.

In some instances, a desired glycosylation pattern will include a low level *(e.g.,* less than about 20%, about 15%, about 10%, about 5%, about 1%, or less) or high level (e.g., greater than about 60%, about 65%, about 70%, about 75%, about 80%, about 85%, about 90%, about 95%, about 98%, about 99%, or more) of a sialic acid linked to an *N*-acetylglucosamine.

In some instances, a desired glycosylation pattern will include a low level *(e.g.,* less than about 20%, about 15%, about 10%, about 5%, about 1%, or less) or high level (e.g., greater than about 60%, about 65%, about 70%, about 75%, about 80%, about 85%, about 90%, about 95%, about 98%, about 99%, or more) of an acetylated glycan.

Whether or not monitoring production of a particular target protein for quality control purposes, the present disclosure may be utilized, for example, to monitor glycosylation at particular stages of development, or under particular growth conditions.

In some particular instances of the present disclosure methods described herein can be used to characterize and/or control or compare the quality of therapeutic products. To give but one example, the present methodologies can be used to assess glycosylation in cells producing a therapeutic protein product. Particularly given that glycosylation can often affect the activity, bioavailability, or other characteristics of a therapeutic protein product, methods for assessing cellular glycosylation during production of such a therapeutic protein product are particularly desirable. Among other things, the present disclosure can facilitate real time analysis of glycosylation in production systems for therapeutic proteins.

Representative therapeutic glycoprotein products whose production and/or quality can be monitored in accordance with the present disclosure include, for example, any of a variety of hematologic agents (including, for instance, erythropoietins, blood-clotting factors, etc.), interferons, colony stimulating factors, antibodies, enzymes, and hormones.

Representative commercially available glycoprotein products include, for example:

| **Protein Product** | **Reference Drug** |
|---|---|
| interferon gamma-1b | Actimmune^{®} |
| alteplase; tissue plasminogen activator | Activase^{®}/Cathflo^{®} |
| Recombinant antihemophilic factor | Advate |
| human albumin | Albutein^{®} |
| laronidase | Aldurazyme^{®} |
| interferon alfa-N3, human leukocyte derived | Alferon N^{®} |
| human antihemophilic factor | Alphanate^{®} |
| virus-filtered human coagulation factor IX | AlphaNine^{®} SD |
| Alefacept; recombinant, dimeric fusion protein LFA3-Ig | Amevive^{®} |
| bivalirudin | Angiomax^{®} |
| darbepoetin alfa | Aranesp™ |
| bevacizumab | Avastin™ |
| interferon beta-1 a; recombinant | Avonex^{®} |
| coagulation factor IX | BeneFix™ |
| Interferon beta-1b | Betaseron^{®} |
| Tositumomab | Bexxar^{®} |
| antihemophilic factor | Bioclate™ |
| human growth hormone | BioTropin™ |
| botulinum toxin type A | Botox^{®} |
| alemtuzumab | Campath^{®} |
| acritumomab; technetium-99 labeled | CEA-Scan^{®} |
| alglucerase; modified form of beta-glucocerebrosidase | Ceredase^{®} |
| imiglucerase; recombinant form of beta-glucocerebrosidase | Cerezyme^{®} |
| crotalidae polyvalent immune Fab, ovine | CroFab™ |
| digoxin immune Fab, ovine | DigiFab™ |
| rasburicase | Elitek^{®} |
| etanercept | Enbrel^{®} |
| epoietin alfa | Epogen^{®} |
| cetuximab | Erbitux™ |
| algasidase beta | Fabrazyme^{®} |
| urofollitropin | Fertinex™ |
| follitropin beta | Follistim™ |
| teriparatide | Forteo^{®} |
| human somatropin | GenoTropin^{®} |
| glucagon | GlucaGen^{®} |
| follitropin alfa | Gonal-F^{®} |
| antihemophilic factor | Helixate^{®} |
| Antihemophilic Factor; Factor XIII | Hemofil^{®} |
| insulin | Humalog^{®} |
| antihemophilic factor/von Willebrand factor complex-human | Humate-P^{®} |
| somatotropin | Humatrope^{®} |
| adalimumab | HUMIRA™ |
| human insulin | Humulin^{®} |
| recombinant human hyaluronidase | Hylenex™ |
| interferon alfacon-1 | Infergen^{®} |
| Eptifibatide | Integrilin™ |
| alpha-interferon | Intron A^{®} |
| palifermin | Kepivance |
| anakinra | Kineret™ |
| antihemophilic factor | Kogenate^{®}FS |
| insulin glargine | Lantus^{®} |
| granulocyte macrophage colony-stimulating factor | Leukine^{®}/Leukine^{®} Liquid |
| lutropin alfa, for injection | Luveris |
| OspA lipoprotein | LYMErix™ |
| ranibizumab | Lucentis^{®} |
| gemtuzumab ozogamicin | Mylotarg™ |
| galsulfase | Naglazyme™ |
| nesiritide | Natrecor^{®} |
| pegfilgrastim | Neulasta™ |
| oprelvekin | Neumega^{®} |
| filgrastim | Neupogen^{®} |
| fanolesomab | NeutroSpec™ (formerly LeuTech^{®}) |
| somatropin [rDNA] | Norditropin^{®}/Norditropin Nordiflex^{®} |
| insulin; zinc suspension; | Novolin L^{®} |
| insulin; isophane suspension | Novolin N^{®} |
| insulin, regular; | Novolin R^{®} |
| insulin | Novolin^{®} |
| coagulation factor VIIa | NovoSeven^{®} |
| somatropin | Nutropin^{®} |
| immunoglobulin intravenous | Octagam^{®} |
| PEG-L-asparaginase | Oncaspar^{®} |
| abatacept, fully human soluable fusion protein | Orencia™ |
| muromomab-CD3 | Orthoclone OKT3^{®} |
| human chorionic gonadotropin | Ovidrel^{®} |
| peginterferon alfa-2a | Pegasys^{®} |
| pegylated version of interferon alfa-2b | PEG-Intron™ |
| Abarelix (injectable suspension); gonadotropin-releasing hormone antagonist | Plenaxis™ |
| epoietin alfa | Procrit^{®} |
| aldesleukin | Proleukin, IL-2^{®} |
| somatrem | Protropin^{®} |
| dornase alfa | Pulmozyme^{®} |
| Efalizumab; selective, reversible T-cell blocker | Raptiva™ |
| combination of ribavirin and alpha interferon | Rebetron™ |
| Interferon beta 1a | Rebif^{®} |
| antihemophilic factor | Recombinate^{®} |
| rAHF/ntihemophilic factor | ReFacto^{®} |
| lepirudin | Refludan^{®} |
| infliximab | Remicade^{®} |
| abciximab | ReoPro™ |
| reteplase | Retavase™ |
| rituximab | Rituxan™ |
| interferon alfa-2a | Roferon-A^{®} |
| somatropin | Saizen^{®} |
| synthetic porcine secretin | SecreFlo™ |
| basiliximab | Simulect^{®} |
| eculizumab | Soliris^{®} |
| pegvisomant | Somavert^{®} |
| Palivizumab; recombinantly produced, humanized mAb | Synagis™ |
| thyrotropin alfa | Thyrogen^{®} |
| tenecteplase | TNKase™ |
| natalizumab | Tysabri^{®} |
| human immune globulin intravenous 5% and 10% solutions | Venoglobulin-S^{®} |
| interferon alfa-n1, lymphoblastoid | Wellferon^{®} |
| drotrecogin alfa | Xigris™ |
| Omalizumab; recombinant DNA-derived humanized monoclonal antibody targeting immunoglobulin-E | Xolair^{®} |
| daclizumab | Zenapax^{®} |
| ibritumomab tiuxetan | Zevalin™ |
| Somatotropin | Zorbtive™ (Serostim^{®}) |

In some instances, the disclosure provides methods in which glycans from different sources or samples are compared with one another. In certain instances, the disclosure provides methods used to monitor the extent and/or type of glycosylation occurring in different cell cultures.

In some examples, multiple samples from the same source are obtained over time, so that changes in glycosylation patterns are monitored. In some instances, glycan-containing samples are removed at about 30 second, about 1 minute, about 2 minute, about 5 minute, about 10 minute, about 30 minute, about 1 hour, about 2 hour, about 3 hour, about 4 hour, about 5 hour, about 10 hour, about 12 hour, or about 18 hour intervals, or at even longer intervals. In some instances, glycan-containing samples are removed at irregular intervals. In some instances, glycan-containing samples are removed at 5 hour intervals.

In some instances, one of the samples is a historical sample or a record of a historical sample. In some instances, one of the samples is a reference sample.

In some instances, glycans from different cell culture samples prepared under conditions that differ in one or more selected parameters (e.g., cell type, culture type [e.g., continuous feed vs batch feed, etc.], culture conditions [e.g., type of media, presence or concentration of particular component of particular medium(a), osmolarity, pH, temperature, timing or degree of shift in one or more components such as osmolarity, pH, temperature, etc.], culture time, isolation steps, etc.) but are otherwise identical, are compared, so that effects of the selected parameter on N-glycosylation patterns are determined. In certain instances, glycans from different cell culture samples prepared under conditions that differ in a single selected parameter are compared so that effects of the single selected parameter on glycosylation patterns is determined. Among other applications, therefore, use of techniques as described herein may facilitate determination of the effects of particular parameters on glycosylation patterns in cells.

In some instances, glycans from different batches of a glycoprotein of interest (e.g., a therapeutic glycoprotein), whether prepared by the same method or by different methods, and whether prepared simultaneously or separately, are compared. In such instances, the present disclosure facilitates quality control of glycoprotein preparation. Alternatively or additionally, some such instances facilitate monitoring of progress of a particular culture producing a glycoprotein of interest (e.g., when samples are removed from the culture at different time points and are analyzed and compared to one another). In any of these instances, features of the glycan analysis can be recorded, for example in a quality control record. As indicated above, in some instances, a comparison is with a historical record of a prior, or standard batch and/or with a reference sample of glycoprotein.

In certain instances, the present disclosure may be utilized in studies to modify the glycosylation characteristics of a cell, for example to establish a cell line and/or culture conditions with one or more desirable glycosylation characteristics. Such a cell line and/or culture conditions can then be utilized, if desired, for production of a particular target glycoconjugate (e.g., glycoprotein) for which such glycosylation characteristic(s) is/are expected to be beneficial.

In certain instances, techniques of the present disclosure are applied to glycans that are present on the surface of cells. In some such instances, the analyzed glycans are substantially free of non-cell-surface glycans. In some such instances, the analyzed glycans, when present on the cell surface, are present in the context of one or more cell surface glycoconjugates (e.g., glycoproteins or glycolipids).

In some particular instances, cell surface glycans are analyzed in order to assess glycosylation of one or more target glycoproteins of interest, particularly where such target glycoproteins are not cell surface glycoproteins. Such instances can allow one to monitor glycosylation of a target glycoprotein without isolating the glycoprotein itself. In certain instances, the present disclosure provides methods of using cell-surface glycans as a readout of or proxy for glycan structures on an expressed glycoprotein of interest. In certain instances, such methods include, but are not limited to, post process, batch, screening or "in line" measurements of product quality. Such methods can provide for an independent measure of the glycosylation pattern of a produced glycoprotein of interest using a byproduct of the production reaction (e.g., the cells) without requiring the use of destruction of any produced glycoprotein. Furthermore, methods of the present disclosure can avoid the effort required for isolation of product and the potential selection of product glycoforms that may occur during isolation.

In certain instances, techniques of the present disclosure are applied to glycans that are secreted from cells. In some such instances, the analyzed glycans are produced by cells in the context of a glycoconjugate (e.g., a glycoprotein or glycolipid).

According to the present disclosure, techniques described herein can be used to detect desirable or undesirable glycans, for example to detect or quantify the presence of one or more contaminants in a product, or to detect or quantify the presence of one or more active or desired species.

In various instances the methods can be used to detect biomarkers indicative of, e.g., a disease state, prior to the appearance of symptoms and/or progression of the disease state to an untreatable or less treatable condition, by detecting one or more specific glycans whose presence or level (whether absolute or relative) may be correlated with a particular disease state (including susceptibility to a particular disease) and/or the change in the concentration of such glycans over time.

In certain instances, methods described herein facilitate detection of glycans that are present at very low levels in a source (e.g., a biological sample, glycan preparation, etc.). In such instances, it is possible to detect and/or optionally quantify the levels of glycans that are present at levels less than about 10%, 5%, 4%, 3%, 2%, 1.5%, 1%, 0.75%, 0.5%, 0.25%, 0.1%, 0.075%, 0.05%, 0.025%, or 0.01% within a population of glycans. In some instances, it is possible to detect and/or optionally quantify the levels of glycans comprising between 0.1% and 5%, e.g., between 0.1% and 2%, e.g., between 0.1 % and 1% of a glycan preparation. In certain instances, it is possible to detect and/or optionally quantify the levels of cell surface glycans at between about 0.1 fmol to about 1 mmol.

In some instances, methods described herein allow for detection of particular linkages that are present at low levels within a population of glycans. For example, the present methods allow for detection of particular linkages that are present at levels less than 10%, less than 5%, less than 4%, less than 3%, less than 2%, less than 1.5%, less than 1%, less than 0.75%, less than 0.5%, less than 0.25%, less than 0.1%, less than 0.075%, less than 0.05%, less than 0.025%, or less than 0.01% within a population of glycans.

In some instances, methods described herein allow for detection of relative levels of individual glycan species within a population of glycans. For example, the area under each peak of a liquid chromatograph can be measured and expressed as a percentage of the total. Such an analysis provides a relative percent amount of each glycan species within a population of glycans.

In some instances, techniques described herein may be combined with one or more other technologies for the detection, analysis, and or isolation of glycans or glycoconjugates. For example, in certain instances, glycans are analyzed in accordance with the present disclosure using one or more available methods (to give but a few examples, see Anumula, Anal. Biochem. 350(1):1,2006; Klein et al., Anal. Biochem., 179:162, 1989; and/or Townsend, R.R. Carbohydrate Analysis" High Performance Liquid Chromatography and Capillary Electrophoresis., Ed. Z. El Rassi, pp 181-209, 1995). For example, in some instances, glycans are characterized using one or more of chromatographic methods, electrophoretic methods, nuclear magnetic resonance methods, and combinations thereof. Exemplary such methods include, for example, NMR, mass spectrometry, liquid chromatography, 2-dimensional chromatography, SDS-PAGE, antibody staining, lectin staining, monosaccharide quantitation, capillary electrophoresis, fluorophore-assisted carbohydrate electrophoresis (FACE), micellar electrokinetic chromatography (MEKC), exoglycosidase or endoglycosidase treatments, and combinations thereof. Those of ordinary skill in the art will be aware of other methods that can be used to characterize glycans together with the IMAC methods described herein.

In some instances, glycan structure and composition can be analyzed by chromatographic methods, including but not limited to, liquid chromatography (LC), high performance liquid chromatography (HPLC), ultra performance liquid chromatography (UPLC), thin layer chromatography (TLC), amide column chromatography, and combinations thereof.

In some instances, glycan structure and composition can be analyzed by mass spectrometry (MS) and related methods, including but not limited to, tandem MS, LC-MS, LC-MS/MS, matrix assisted laser desorption ionisation mass spectrometry (MALDI-MS), Fourier transform mass spectrometry (FTMS), ion mobility separation with mass spectrometry (IMS-MS), electron transfer dissociation (ETD-MS), and combinations thereof.

In some instances, glycan structure and composition can be analyzed by electrophoretic methods, including but not limited to, capillary electrophoresis (CE), CE-MS, gel electrophoresis, agarose gel electrophoresis, acrylamide gel electrophoresis, SDS-polyacrylamide gel electrophoresis (SDS-PAGE) followed by Western blotting using antibodies that recognize specific glycan structures, and combinations thereof.

In some instances, glycan structure and composition can be analyzed by nuclear magnetic resonance (NMR) and related methods, including but not limited to, one-dimensional NMR (1D-NMR), two-dimensional NMR (2D-NMR), correlation spectroscopy magnetic-angle spinning NMR (COSY-NMR), total correlated spectroscopy NMR (TOCSY-NMR), heteronuclear single-quantum coherence NMR (HSQC-NMR), heteronuclear multiple quantum coherence (HMQC-NMR), rotational nuclear overhauser effect spectroscopy NMR (ROESY-NMR), nuclear overhauser effect spectroscopy (NOESY-NMR), and combinations thereof.

The present disclosure will be more specifically illustrated by the following example. However, it should be understood that the present disclosure is not limited by this example in any manner.

### Example

### Example: Use of IMAC to Differential Phosphorylated from Unphosphorylated Glycans

A 1:1 mixture of mannose and mannose-6-phosphate was applied to a Ga³⁺ IMAC column, followed by a washing step. Loading and washing conditions were as described in, for example, Posewitz & Tempst, Anal. Chem. 71:2883. Mannose did not bind to the resin and mannose-6-phosphate was eluted with 100 mM ammonium bicarbonate.

The fractions were dried and labeled with 2-aminobenzamide. Labeled glycans were separated from excess label by paper chromatography. Labeled fractions were then run on normal phase HPLC for analysis.

As is illustrated in Figure 1, mannose did not bind to the resin, but phosphorylated mannose was retained. The present disclosure therefore demonstrates that IMAC is an effective tool that can be applied to glycan samples to separate phosphorylated glycans from non-phosphorylated glycans (e.g., high mannose structures). According to the present disclosure, this procedure is also applicable to glycoconjugates and will separate conjugates of phosphorylated glycans from other components of a sample, and particularly from other glycan-containing components.

## Claims

1. A method of separating at least two glycans present in a glycan preparation, the method comprising steps of:
(i) providing a glycan preparation that includes at least two glycans with differences in phosphorylation, wherein the at least two glycans have each been cleaved or otherwise released from a glycoconjugate;
(ii) contacting the glycan preparation with a resin to which metal ions are immobilized under conditions that cause the at least two glycans to exhibit differing affinities for the resin as a result of the differences in phosphorylation; and
(iii) separating the at least two glycans based on their differing affinities for the resin.

2. The method of claim 1, further comprising a step of:
(iv) washing the resin-containing glycans with at least one solvent.

3. The method of claim 1 or claim 2, further comprising a step of:
(v) eluting retained glycans from the resin.

4. The method of claim 3, wherein the step of eluting comprises eluting with a solvent different from any solvent used for washing.

5. The method of claim 1, wherein:
the step of providing comprises providing a glycan preparation that includes at least one phosphorylated glycan and at least one sulfated glycan; and
the step of contacting comprises contacting the glycan preparation with a resin to which metal ions are immobilized under conditions that cause the at least one phosphorylated glycan to be retained on the resin differently from the sulfated glycan.

6. The method of claim 1, wherein:
the step of providing comprises providing a glycan preparation that includes at least one phosphorylated glycan and at least one sialylated glycan; and
the step of contacting comprises contacting the glycan preparation with a resin to which metal ions are immobilized under conditions that cause the at least one phosphorylated glycan to be retained on the resin differently from the sialylated glycan.

7. The method of claim 1, wherein the glycan preparation is obtained from a glycopeptide preparation.

8. The method of claim 1 wherein the step of providing a glycan preparation comprises releasing glycans from a biological sample.

9. The method of claim 8 wherein the biological sample is selected from the group consisting of samples from tissue culture, bioreactors, human or animal tissue, plants, fruits, vegetables, single-celled microorganisms, and multicellular organisms.

10. The method of claim 8 wherein the biological sample is selected from the group consisting of blood, plasma, serum, urine, bile, seminal fluid, cerebrospinal fluid, aqueous or vitreous humor, or any bodily secretion, a transudate, an exudate, and fluid obtained from a joint.

11. A method of analyzing a sample containing glycans, the method comprising steps of:
cultivating a cell that produces a plurality of glycoconjugates;
obtaining a glycan preparation from said plurality of glycoconjugates, wherein the glycan preparation includes glycans having differences in phosphorylation and wherein said glycans have been cleaved or otherwise released from said plurality of glycoconjugates; and
analyzing a sample containing the glycans by contacting the sample with a resin to which metal ions have been immobilized, the contacting being performed under conditions such that individual glycans are differentially retained on the resin as a result of the differences in phosphorylation.

12. The method of claim 11 wherein the step of obtaining comprises exposing the cell to at least one procedure selected from the group consisting of protease treatment, glycosidase treatment, fractionation, separation, labeling, and combinations thereof.

## Patentansprüche

1. Verfahren zur Trennung von mindestens zwei in einer Glykanzubereitung vorhandenen Glykanen, wobei das Verfahren die folgenden Schritte umfasst:
(i) die Bereitstellung einer Glykanzubereitung mit mindestens zwei unterschiedlich phosphorylierten Glykanen, wobei die mindestens zwei Glykane jeweils von einem Glykokonjugat abgespalten oder anderweitig freigesetzt wurden,
(ii) das Inkontaktbringen der Glykanzubereitung mit einem Harz, an dem Metallionen immobilisiert sind, unter Bedingungen, die die mindestens zwei Glykane dazu bringen, als Folge der Unterschiede bei der Phosphorylierung unterschiedliche Affinitäten für das Harz zu zeigen, und
(iii) die Trennung der mindestens zwei Glykane, basierend auf ihren unterschiedlichen Affinitäten zum Harz.

2. Verfahren nach Anspruch 1, welches weiterhin den folgenden Schritt umfasst:
(iv) das Waschen der harzhaltigen Glykane mit mindestens einem Lösungsmittel.

3. Verfahren nach Anspruch 1 oder Anspruch 2, welches weiterhin den folgenden Schritt umfasst:
(v) das Eluieren zurückgehaltener Glykane vom Harz.

4. Verfahren nach Anspruch 3, wobei der Elutionsschritt ein Eluieren mit einem von einem zum Waschen verwendeten Lösungsmittel verschiedenen Lösungsmittel umfasst.

5. Verfahren nach Anspruch 1, wobei:
der Schritt der Bereitstellung die Bereitstellung einer Glykanzubereitung umfasst, die mindestens ein phosphoryliertes Glykan und mindestens ein sulfatiertes Glykan umfasst, und
der Schritt des Inkontaktbringens das Inkontaktbringen der Glykanzubereitung mit einem Harz, an dem Metallionen immobilisiert sind, unter Bedingungen, bei denen mindestens ein phosphoryliertes Glykan unterschiedlich vom sulfatierten Glykan auf dem Harz zurückgehalten wird, umfasst.

6. Verfahren nach Anspruch 1, wobei:
der Schritt der Bereitstellung die Bereitstellung einer Glykanzubereitung umfasst, die mindestens ein phosphoryliertes Glykan und mindestens ein sialyliertes Glykan umfasst, und
der Schritt des Inkontaktbringens das Inkontaktbringen der Glykanzubereitung mit einem Harz, an dem Metallionen immobilisiert sind, unter Bedingungen, bei denen mindestens ein phosphoryliertes Glykan unterschiedlich vom sialylierten Glykan auf dem Harz zurückgehalten wird, umfasst.

7. Verfahren nach Anspruch 1, wobei die Glykanzubereitung aus einer Glykopeptidzubereitung erhalten wird.

8. Verfahren nach Anspruch 1, wobei der Schritt der Bereitstellung einer Glykanzubereitung die Freisetzung von Glykanen aus einer biologischen Probe umfasst.

9. Verfahren nach Anspruch 8, wobei die biologische Probe aus der aus Proben von Gewebekulturen, Bioreaktoren, humanem oder tierischem Gewebe, Pflanzen, Früchten, Gemüsen, einzelligen Mikroorganismen und multizellulären Organismen bestehenden Gruppe ausgewählt ist.

10. Verfahren nach Anspruch 8, wobei die biologische Probe aus der aus Blut, Plasma, Serum, Urin, Galle, Samenflüssigkeit, Zerebrospinalflüssigkeit, Kammerwasser bzw. wässrigem Humor, oder einer beliebigen körperlichen Absonderung, einem Transudat, einem Exudat und aus einem Gelenk erhaltenen Flüssigkeit bestehenden Gruppe ausgewählt ist.

11. Verfahren zur Analyse einer Glykane enthaltenden Probe, wobei das Verfahren die folgenden Schritte umfasst:
das Kultivieren einer eine Mehrzahl von Glykokonjugaten produzierenden Zelle;
das Gewinnen einer Glykanzubereitung aus der Mehrzahl von Glykokonjugaten, wobei die Glykanzubereitung unterschiedlich phosphorylierte Glykane umfasst und wobei die Glykane von der Mehrzahl von Glykokonjugaten abgespalten oder anderweitig freigesetzt wurden, und
das Analysieren einer die Glykane enthaltenden Probe durch Inkontaktbringen der Probe mit einem Harz, an dem Metallionen immobilisiert worden sind, wobei das Inkontaktbringen unter Bedingungen erfolgt, bei denen die einzelnen Glykane als Folge der Unterschiede bei der Phosphorylierung unterschiedlich auf dem Harz zurückgehalten werden.

12. Verfahren nach Anspruch 11, wobei der Schritt der Gewinnung das Aussetzen der Zelle gegenüber mindestens einer Vorschrift ausgewählt aus der Gruppe bestehend aus Proteasebehandlung, Glykosidasebehandlung, Fraktionierung, Auftrennung, Markierung und Kombinationen davon umfasst.

## Revendications

1. Procédé de séparation d'au moins deux glycanes présents dans une préparation de glycanes, le procédé comprenant les étapes de :
(i) fourniture d'une préparation de glycanes qui comprend au moins deux glycanes avec des différences de phosphorylation, les au moins deux glycanes ayant chacun été clivés ou autrement libérés d'un glycoconjugué ;
(ii) mise en contact de la préparation de glycanes avec une résine sur laquelle des ions métalliques sont immobilisés dans des conditions qui amènent les au moins deux glycanes à présenter des affinités différentes pour la résine en conséquence des différences de phosphorylation ; et
(iii) séparation des au moins deux glycanes sur la base de leurs affinités différentes pour la résine.

2. Procédé de la revendication 1, comprenant en outre une étape de :
(iv) lavage de la résine contenant des glycanes avec au moins un solvant.

3. Procédé de la revendication 1 ou la revendication 2, comprenant en outre une étape de :
(v) élution des glycanes retenus depuis la résine.

4. Procédé de la revendication 3, dans lequel l'étape d'élution comprend l'élution avec un solvant différent d'un solvant quelconque utilisé pour le lavage.

5. Procédé de la revendication 1, dans lequel :
l'étape de fourniture comprend la fourniture d'une préparation de glycanes qui comprend au moins un glycane phosphorylé et au moins un glycane sulfaté ; et
l'étape de mise en contact comprend la mise en contact de la préparation de glycanes avec une résine sur laquelle des ions métalliques sont immobilisés dans des conditions qui amènent l'au moins un glycane phosphorylé à être retenu sur la résine différemment du glycane sulfaté.

6. Procédé de la revendication 1, dans lequel :
l'étape de fourniture comprend la fourniture d'une préparation de glycanes qui comprend au moins un glycane phosphorylé et au moins un glycane sialylé ; et
l'étape de mise en contact comprend la mise en contact de la préparation de glycanes avec une résine sur laquelle des ions métalliques sont immobilisés dans des conditions qui amènent l'au moins un glycane phosphorylé à être retenu sur la résine différemment du glycane sialylé.

7. Procédé de la revendication 1, dans lequel la préparation de glycanes est obtenue à partir d'une préparation de glycopeptide.

8. Procédé de la revendication 1 dans lequel l'étape de fourniture d'une préparation de glycanes comprend la libération de glycanes à partir d'un échantillon biologique.

9. Procédé de la revendication 8 dans lequel l'échantillon biologique est choisi dans le groupe constitué d'échantillons de culture de tissu, bioréacteurs, tissu humain ou animal, plantes, fruits, légumes, micro-organismes unicellulaires, et organismes pluricellulaires.

10. Procédé de la revendication 8 dans lequel l'échantillon biologique est choisi dans le groupe constitué de sang, plasma, sérum, urine, bile, liquide séminal, liquide cérébrospinal, humeur aqueuse ou vitrée, ou une sécrétion corporelle quelconque, un transsudat, un exsudat, et un liquide obtenu à partir d'une articulation.

11. Procédé d'analyse d'un échantillon contenant des glycanes, le procédé comprenant les étapes de :
culture d'une cellule qui produit une pluralité de glycoconjugués ;
obtention d'une préparation de glycanes à partir de ladite pluralité de glycoconjugués, la préparation de glycanes comprenant des glycanes ayant des différences de phosphorylation et lesdits glycanes ayant été clivés ou autrement libérés de ladite pluralité de glycoconjugués ; et
analyse d'un échantillon contenant les glycanes par mise en contact de l'échantillon avec une résine sur laquelle des ions métalliques ont été immobilisés, la mise en contact étant effectuée dans des conditions telles que des glycanes individuels sont retenus de façon différentielle sur la résine en conséquence des différences de phosphorylation.

12. Procédé de la revendication 11 dans lequel l'étape d'obtention comprend l'exposition de la cellule à au moins une procédure choisie dans le groupe constitué d'un traitement par une protéase, un traitement par une glycosidase, un fractionnement, une séparation, un marquage, et des combinaisons de ceux-ci.
